# EUROPEAN PATENT APPLICATION

(11) **EP 2 559 680 A1**
(43) Date of publication of application: **20.02.2013**
(21) Application number: 11250728.0
(22) Date of filing: 16.08.2011
(51) Int. Cl.: C07C 51/12, C07C 67/36, C07C 53/08, C07C 69/14

(54) **Carbonylation of methanol**

(71) Applicant: BP Chemicals Limited, Sunbury-on-Thames, Middlesex TW16 7BP (GB)
(72) Inventor: Gaemers, Sander, HU12 8DS, Hull, East Yorkshire (GB); Law, David John, HU12 8DS, Hull, East Yorkshire (GB); Sunley, John Glenn, HU12 8DS, Hull, East Yorkshire (GB)
(74) Representative: Wilson, Nicola Ann

(57) **Abstract**

A carbonylation process for the production of acetic acid and/or methyl acetate by contacting, in a carbonylation reactor, carbon monoxide with a feed comprising methanol and/or reactive derivative thereof in the vapour phase and in the presence of a heterogeneous catalyst comprising a heteropolyacid, wherein the heteropolyacid has been ion-exchanged or loaded with an organometallic complex of a group VIII metal and at least one ligand, which ligand comprises one or more nitrogen and/or phosphorous donor atoms.

## Description

The present invention relates to a carbonylation process for the production of acetic acid and or methyl acetate by contacting carbon monoxide with a feed comprising methanol and/or reactive derivative thereof in the vapour phase using a heterogeneous catalyst.

The production of acetic acid by the rhodium-catalysed or iridium-catalysed, iodide-promoted carbonylation of methanol in a homogeneous liquid-phase reaction medium is well-known and is operated on a commercial scale. The desirability of employing heterogeneous carbonylation catalysts for the purpose of facilitating product separation from the catalyst has also been recognised. Heterogeneous carbonylation catalysts comprising heteropolyacids are described in a number of patent publications including, for example EP 0353722 A2, US 6,127,432, EP 1720819 and EP 1828093.

EP 0353722 A2 describes a process for the vapour phase carbonylation of one or more alcohols, ethers or ether alcohols to esters and, optionally, to carboxylic acids over a solid catalyst comprising a polyoxometalate anion in which the metal is at least one taken from Group V and VI of the periodic table, such as Mo, W, V, Nb, Cr and Ta, complexed with at least one Group VIIIA cation, such as Fe, Ru, Os, Co, Rh, Ir, Ni, Pd and Pt.

US 6,127,432 describes processes for the conversion of a feedstock comprising carbon monoxide and hydrogen to a product stream comprising at least one of an ester, acid, acid anhydride and mixtures thereof. US 6,127,432 also describes a process for converting an alcohol, ether and/or ether alcohol to oxygenated products such as esters, acids, acid anhydrides and mixtures thereof, which process may be conducted in the vapour phase over a heterogeneous alcohol carbonylation catalyst selected from a solid superacid, clay, zeolite or molecular sieve. The alcohol carbonylation catalysts include heteropolyacids comprising a polyoxometalate anion in which a metal, or mixture of metals, selected from Groups 4, 5, 6 and 7 metals is complexed with a cation from a member of Group 7, 8, 9 10 and/or 11 metals, such as Fe, Ru, Os, Co, Rh, Ir, Ni, Pd and Pt. A preferred heteropolyacid comprises MW₁₂PO₄₀, wherein M is Ir, Ru, Rh, Pd and combinations thereof. US 6,127,432 states that the stability of the heterogeneous alcohol carbonylation catalyst is improved by use of hydrogen or a feedstock containing hydrogen in the carbonylation process.

EP 1720819 describes a carbonylation process for producing a carbonylation product by contacting carbon monoxide with a feed comprising an alcohol and/or a reactive derivative thereof in the vapour phase using an heterogeneous heteropolyacid catalyst comprising one or more metal cations selected from Cu, Fe, Ru, Os, Co, Rh, Ir, Ni, Pd and Pt and wherein there is at least 0.5wt% water present in the feed.

EP 1828093 describes a carbonylation process for the production of a carbonylation product such as a carboxylic acid and a carboxylic acid ester by contacting carbon monoxide with a feed comprising an alcohol such as methanol and/or a reactive derivative thereof such as methyl acetate in the vapour phase using a heterogeneous heteropolyacid catalyst which has been ion-exchanged or loaded with at least one metal selected from rhodium, iridium, copper and palladium and a Group IA metal selected from lithium, sodium, potassium and rubidium.

It has now been found that the use of a heterogeneous catalyst comprising a heteropolyacid, wherein the heteropolyacid has been ion-exchanged or loaded with an organometallic complex of a group VIII metal and at least one ligand, which ligand comprises one or more nitrogen and/or phosphorous donor atoms, provides an improved carbonylation process. For example, catalyst activity and/or lifetime may be improved.

Accordingly, the present invention provides a carbonylation process for the production of acetic acid and/or methyl acetate by contacting, in a carbonylation reactor, carbon monoxide with a feed comprising methanol and/or reactive derivative thereof in the vapour phase and in the presence of a heterogeneous catalyst comprising a heteropolyacid, wherein the heteropolyacid has been ion-exchanged or loaded with an organometallic complex of a group VIII metal and at least one ligand, which ligand comprises one or more nitrogen and/or phosphorous donor atoms.

Heteropolyacids are well known. Typically, the heteropolyacid anion comprises 2 to 18 oxygen-linked polyvalent metal atoms, which are known in the art as peripheral atoms. These peripheral atoms surround one or more central atoms in a symmetrical manner. The peripheral atoms are usually one or more of molybdenum, tungsten, vanadium, niobium, chromium and tantalum, but may be or may include other metals. The central atoms are usually silicon or phosphorus but can comprise any one of a large variety of atoms from Groups I-VIII in the Periodic Table of elements. These include, for instance, cupric ions; divalent beryllium, zinc, cobalt or nickel ions; trivalent boron, aluminium, gallium, iron, cerium, arsenic, antimony, phosphorus, bismuth, chromium or rhodium ions; tetravalent silicon, germanium, tin, titanium, zirconium, vanadium, sulphur, tellurium, manganese nickel, platinum, thorium, hafnium, cerium ions and other rare earth ions; pentavalent phosphorus, arsenic, vanadium, antimony ions; hexavalent tellurium ions; and heptavalent iodine ions. Such heteropolyacids are also known as "polyoxoanions", "polyoxometallates" or "metal oxide clusters". The structures of some of the well known anions are named after the original researchers in this field such as, for example, the structures known as Keggin, Wells-Dawson and Anderson-Evans-Perloff structures.

Heteropolyacids may be represented by the formula H₃M₁₂XO₄₀ where M is tungsten, molybdenum, chromium, vanadium, tantalum or niobium and X is phosphorous, or by the formula H₄M₁₂XO₄₀ where M is tungsten, molybdenum, chromium, vanadium, tantalum or niobium and X is silicon.

Preferably, the heteropolyacid is selected from silicotungstic acids, silicomolybdic acids, phosphotungstic acids, phosphomolybdic acids, such as the following heteropolyacids :
12-tungstophosphoric acid H₃[PW₁₂O₄₀].xH₂O
12-molybdophosphoric acid H₃[PMo₁₂O₄₀].xH₂O
12-tungstosilicic acid H₄[SiW₁₂O₄₀].xH₂O
12-molybdosilicic acid H₄[SiMo₁₂O₄₀].xH₂O

The heteropolyacid of the catalyst used in the process of the present invention is a heteropolyacid which has been ion-exchanged or otherwise loaded with an organometallic complex of a group VIII metal and at least one ligand, which ligand comprises one or more nitrogen and/or phosphorous donor atoms. The at least one Group VIII metal is preferably selected from rhodium, palladium, iridium, and nickel and mixtures thereof, more preferably selected from rhodium, iridium and palladium. Most preferably, the Group VIII metal is rhodium.

The heteropolyacid may optionally be ion-exchanged or loaded with one or more further metals, in addition to the at least one Group VIII metal of the organometallic complex, for example, other transition metals, for example, copper or silver, alkali metals and/or alkaline earth metals, for example, lithium, sodium, potassium, rubidium, and cesium.

The total amount of metal (that is the total of the amount of Group VIII metals present as part of the organometallic complex and the amount of further metals) loaded or ion-exchanged onto the heteropolyacid can vary depending on the heteropolyacid used. Suitably, however, the total amount of metal loaded onto the heteropolyacid should be such that some acidity is retained by the heteropolyacid, for example at least 0.5 protons. Thus, where 12-tungstophosphoric acid H₃[PW₁₂O₄₀] is employed, up to 2.5 protons may be exchanged for metals and where 12-tungstosilicic acid H₄[SiW₁₂O₄₀] is used, up to 3.5 protons can be exchanged by metals.

The catalyst is preferably supported on an inert support. Suitably, the support may be selected from oxide supports such as silica, silica /aluminas, zeolites, clays, diatomaceous earths, titania and alumina. Other non-oxide supports that can be used include silicon carbide, organic polymers such as crosslinked polystyrenes and carbons. Preferably, the support is silica. The support, such as a siliceous support, is suitably in the form of granules, beads, globules, extrudates or pellets.

Where the catalyst is supported the catalyst is typically present at a loading of 10-80% by weight of the total weight of the supported catalyst, that is, the catalyst forms 10-80% by weight of the total weight of the catalyst and the support. Preferably, where the catalyst is supported, the catalyst is present at a loading of 30-70% by weight of the total weight of the supported catalyst.

The organometallic complex of the catalyst used in the process of the present invention comprises at least one ligand, which ligand comprises one or more nitrogen and/or phosphorous donor atoms. The ligand may be a monodentate ligand comprising either a single nitrogen donor atom or a single phosphorous donor atom. Alternatively, the ligand may be polydentate, i.e. comprising two or more donor atoms. For example, the ligand may be bidentate or tridentate. Where the ligand is polydentate, at least one of the donor atoms is a nitrogen or phosphorous donor atom.

Suitable monodentate ligands include triphenylphosphine, biphenylphosphine [BIPHEP] and bis(2-methoxyphenyl)(phenyl)phosphine [P(oMeO-Ph)₂Ph].

Where the ligand is polydentate, all of the donor atoms may be nitrogen atoms, phosphorous atoms or a mixture of nitrogen and phosphorous atoms. Alternatively, where the ligand is polydentate, in addition to one or more nitrogen and/or phosphorous donor atoms, the ligand may comprise further donor atoms, for example, carbon, sulfur and/or oxygen donor atoms.

Preferred ligands suitable for use in the present invention include bidentate and tridentate diphenylphosphino-containing ligands.

Suitable bidentate diphenylphosphino-containing ligands include 1,3-bis(diphenylphosphino)propane [dppp], 1,4-bis(diphenylphosphino)butane [dppb], 1,2-bis(diphenylphosphino)benzene [dpp-Benz], 2,2'-bis(diphenylphosphino)-1,1'-binapthyl [BINAP], 1,2 - bis(diphenylphosphino)ethane [dppe], and the perfluorinated analogue of dppe [dppe(F20)] having the structures shown below:

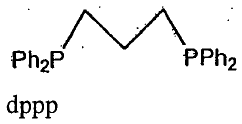

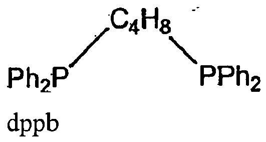

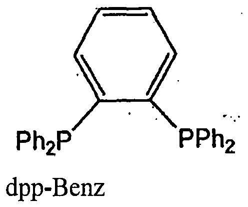

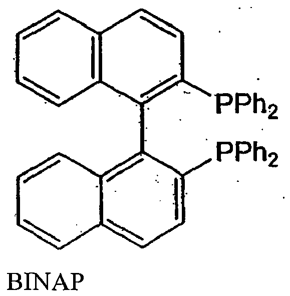

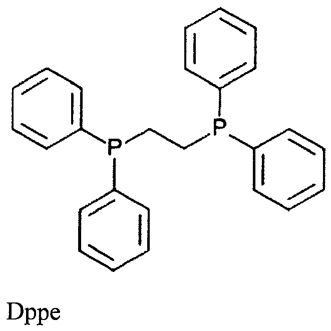

Suitable tridentate diphenylphosphino-containing ligands include 1,1,1-tris(diphenylphosphinomethyl)ethane [TERPHOS] and ((phenylphosphinediyl)bis(ethane-2,1-diyl))bis(diphenylphosphine) [TRIPHOS] and 1,8-bis(diphenylphosphino)anthracene [ANTHRAPHOS] having the structures shown below: 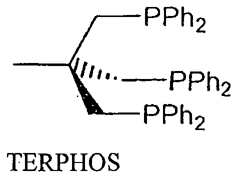 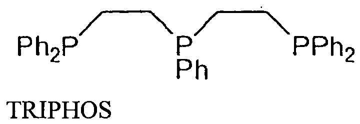 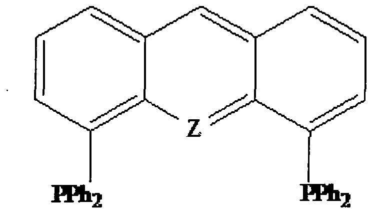 ANTHROPHOS (Z = a carbon donor atom)

Diphenylphosphino-containing ligands which may be bidentate or tridentate ligands (i.e. the ligands have three available donor atoms but in some circumstances may coordinate via only 2 of them) include (9,9-dimethyl-9H-xanthene-4,5-diyl)bis(diphenylphosphine) [Xantphos] and (Oxydi-2,1-phenylene)bis(diphenylphosphine) [DPE-phos] having the structures shown below. Each of Xanthphos and DPE-phos may be bidentate where they coordinate via the 2 phosphorous donor atoms only, or tridentate where they coordinate via the two phosphorus donor atoms and the oxygen donor atom.

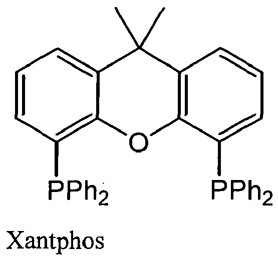

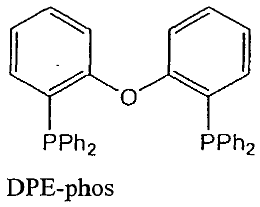

A bidentate nitrogen containing ligand suitable for use in the present invention is 2,2'-bipyridine [bipy] having the structure shown below:

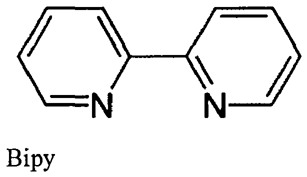

A tridentate nitrogen containing ligand suitable for use in the present invention is 2,2':6',2-terpyridine [TERPY] having the structure shown below:

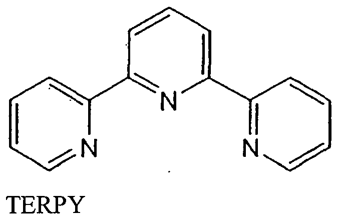

In one embodiment of the process of the present invention, the organometallic complex of the catalyst may comprise one or more further ligands in addition to the at least one ligand comprising one or more nitrogen and/or phosphorous donor atoms. The one or more further ligands may also comprise one or more nitrogen and/or phosphorous atoms. Alternatively, the one or more further ligands may comprise different donor atoms, for example, carbon, sulfur and/or oxygen donor atoms.

The catalyst used in the process of the present invention comprises heteropolyacid, wherein the heteropolyacid has been ion-exchanged or loaded with an organometallic complex of a group VIII metal and at least one ligand, which ligand comprises one or more nitrogen and/or phosphorous donor atoms, as described above. Without wishing to be bound by theory, it is believed that in the organometallic complex the at least one ligand coordinates to (forms a dative bond with) the Group VIII metal via at least one donor atom comprising nitrogen or phosphorous.

The organometallic complex may be prepared, for example, by combining a suitable Group VIII metal compound with a ligand in an appropriate solvent, to form a solution of the organometallic compound. In one embodiment, the catalyst employed in the process of the present invention may be prepared, for example, by combining such a solution of the organometallic compound with a solution of an heteropolyacid in the same solvent, followed by removal of the solvent from the mixture.

According to the present invention the feed which is carbonylated to produce acetic acid or methyl acetate comprises methanol and/or a reactive derivative thereof. Reactive derivatives of methanol which may be used as an alternative to, or in addition to, methanol include methyl acetate, dimethyl ether and methyl iodide.

Water may be produced during the carbonylation process as a by-product of esterification and/or etherification. This water may be recycled to the reactor.

It may be necessary to add "fresh" water to the carbonylation reaction feed in addition to any water that may be recycled in order to maintain the desired concentration of water in the feed to the reactor.

Preferably, at least 0.5wt% water is present in the feed to the carbonylation process. The water may be fresh water and/or recycled water. More preferably, the water (fresh and/or recycle) in the feed to the carbonylation process is present at a concentration of at least 1wt%, such as at least 2wt%. Most preferably, the water in the feed to the carbonylation process is present at a concentration of at least 5wt%.

Preferably, the water (fresh and/or recycle) in the feed to the carbonylation process is present at a concentration of up to 20wt% water, such as up to 15wt% water. Most preferably, the water in the feed to the carbonylation process is present at a concentration of 5 to 15wt%.

The carbon monoxide reactant may be essentially pure or may contain impurities such as carbon dioxide, methane, nitrogen, noble gases and C₁ to C₄ paraffinic hydrocarbons.

The carbon monoxide (CO) may be present in the reaction at any suitable partial pressure, such as a partial pressure of at least 0.1 bar. More particularly, the CO may be fed to the reactor in a suitable molar ratio to the methanol and/or reactive derivative feed, preferably at a CO to methanol and/or reactive derivative molar ratio of at least 1:1, such as at least 5:1, and/or up to 20:1, most preferably in the range 5:1 to 15:1.

In a preferred embodiment of the present invention, the carbonylation reaction may be carried out in the presence of hydrogen. Where the carbonylation reaction is carried out in the presence of hydrogen, hydrogen may be fed to the reactor as an essentially pure hydrogen feed or the hydrogen feed stream may contain impurities, such as carbon oxides and nitrogen. The hydrogen, when present, may be present in the reactor at any suitable hydrogen concentration, such as at a partial pressure of at least 0.1 bar, and is especially fed, either separately from or combined with carbon monoxide, such that the hydrogen to carbon monoxide molar ratio in the reactor is at least 1:20, such as 1:20 to 20:1, most preferably in the range 1:10 to 10:1.

Where hydrogen is used in the process of the present invention, it is especially desirable to use synthesis gas as a source of both the hydrogen and the carbon monoxide. Synthesis gas is commercially produced by the steam reforming of hydrocarbons and by the partial oxidation of hydrocarbons. The separation of carbon monoxide and hydrogen is generally an expensive process to perform and typically involves separation by cryogenic techniques. Advantageously, for use in the process of the present invention it is not necessary to separate hydrogen from carbon monoxide. Further, the use of synthesis gas as the source of both the hydrogen and carbon monoxide allows the process of the present invention to be more easily integrated with a methanol manufacture which uses syngas to make methanol.

Small amounts of carbon dioxide may additionally be present in the feed to the carbonylation process, for example, as a component of synthesis gas.

The process of the invention may be operated at below atmospheric pressure, but is preferably operated at a total pressure in the range from 1 to 100 barg, preferably from 1 to 20 barg.

The process is suitably performed at a temperature in the range from 100 to 300°C, the practical upper operating temperature being dependant on the thermal stability of the catalyst. Preferably the temperature is in the range 150 to 250°C, most preferably in the range 170 to 230°C.

The process is suitably performed by contacting the reactants with the catalyst at a gas hourly space velocity (GHSV) in the range from 100 to 10000 h⁻¹, preferably the GHSV is in the range 500 to 5000 h⁻¹.

In one embodiment, the process of the present invention may be carried out by feeding methanol vapour, dimethyl ether vapour, methyl acetate vapour or mixtures thereof and carbon monoxide gas to a carbonylation reactor containing a fixed or fluidised bed of the catalyst.

The process of the present invention may be operated as a batch or continuous process, preferably as a continuous process.

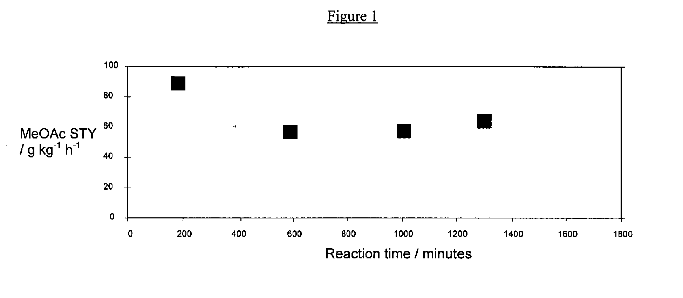

The invention will now be illustrated by reference to the following examples and by reference to figure 1. Figure 1 is a graph showing the space time yield to methyl acetate product in grams per kilogram of catalyst per hour for Example 1.

### Catalyst Preparation

### Catalyst 1

20.10790 g (6.99 mmol) silicotungstic acid was dissolved in 100 ml of tetrahydrofuran (THF). 20.0458 g of silica (Grace, grade G57) was then added and the resulting mixture stirred for 3 hours. 1.2 ml of a 0.059moldm⁻³ aqueous cesium acetate solution was then added to the mixture, followed by 0.5737g (3.44 mmol) silver acetate. The mixture was then stirred in darkness for 1 hour.

A solution of a rhodium-Terpy organometallic complex was formed by adding 20 ml of THF to a mixture of 0.8948g (3.47 mmol) dicarbonyl-acetylacetonato-rhodium(I) and 0.8124g (3.48 mmol) Terpy ligand. Carbon monoxide evolved from the solution.

The solution of the rhodium-Terpy complex was then added to the heteropolyacid/silica mixture and the resulting mixture stirred for 15 minutes. The solvent was then removed from the mixture using a rotary evaporator. The resulting purple powder was dried overnight at 60°C under a stream of nitrogen. The dried powder was then pressed, crushed and sieved to a particle size of 0.5-1 mm in diameter.

The composition of catalyst A was (Rh-Terpy)_{0.5}Ag_{0.5}Cs_{0.1}SiW₁₂ on silica.

### Catalyst 2

20.017 g (6.95 mmol) H₃PW₁₂O₄₀ was dissolved in 100 ml of tetrahydrofuran (THF). 20.008 g of silica (Grace, grade G57) was then added and the resulting mixture stirred for 4 hours.

A solution of a rhodium-bis(diphenylphosphino)propane organometallic complex was formed by adding 20 ml of THF to a mixture of 1.811 g (7.02 mmol) dicarbonylacetylacetonato-rhodium(I) and 3.024g (7.33 mmol) bis(diphenylphosphino)propane ligand.

The solution of the rhodium-bis(diphenylphosphino)propane complex was then added to the heteropolyacid/silica mixture and the resulting mixture stirred for 15 minutes.

The solvent was then removed from the mixture using a low-pressure rotary evaporator. The resulting pale brown powder was dried at 60°C under a stream of nitrogen. The dried powder was then pressed, crushed and sieved to a particle size of 0.5-1 mm in diameter.

The composition of catalyst B was [(dppp)Rh]PW₁₂(H₂) on silica.

### Catalyst 3

19.95 g (6.93 mmol) silicotungstic acid was dissolved in 100 ml of tetrahydrofuran (THF). 19.95 g of silica (Grace, grade G57) was then added and the resulting mixture stirred for 3 hours. 1.0 ml of a 0.071 moldm⁻³ aqueous cesium acetate solution was then added to the mixture.

A solution of a rhodium-dpp-benz complex was formed by adding 20 ml of THF to a mixture of 0.8924g (3.46 mmol) dicarbonyl-acetylacetonato-rhodium(I) and 1.5539g (3.48 mmol) dpp-benz ligand. Carbon monoxide evolved from the solution.

The solution of the rhodium-dpp-benz complex was then added to the heteropolyacid/silica mixture and the resulting mixture stirred for 15 minutes. The solvent was then removed from the mixture using a rotary evaporator. The resulting pale yellow powder was dried overnight at 60°C under a stream of nitrogen. The dried powder was then pressed, crushed and sieved to a particle size of 0.5-1 mm in diameter.

The composition of catalyst C was (Rh-dpp-benz)₀.₅Cs_{0.01}SiW₁₂ on silica.

### Example 1

The catalyst testing procedure employed in Example 1 is set out below.

5ml of catalyst 1 diluted with 15 ml of silica was charged to a quartz tube reactor having a 19 mm diameter and with a supportive frit positioned in the middle of the tube. The reactor was then filled with glass wool above the catalyst. The remainder of the top section of the reactor was packed with carborundum to minimise the dead volume in the reactor. The reactor was positioned in the middle of a vertical furnace, with insulating lagging at the top and bottom of the furnace. The furnace was heated gradually to 100°C. To remove the majority of the water from the catalyst, the furnace remained at 100°C for 20 minutes while nitrogen or carbon monoxide at a gas flow rate of 150 ml/min (GHSV = 1800 h⁻¹) was fed into the top of the reactor. The furnace was then heated gradually to 200°C. After the furnace had reached 200°C, the nitrogen feed (if used) is switched off and replaced by a feed of carbon monoxide at a gas flow rate of 150 ml/min (GHSV = 1800 h^{- 1}). The system was left at temperature until it had fully equilibrated and then methanol was fed to the top of the reactor via a syringe pump such that the molar ratio of carbon monoxide to methanol fed to the reactor was 9 : 1. The liquid and gaseous products pass down through the reactor and into a liquid trap. The liquid trap comprised a coil condenser immersed in water, where the liquid products were collected. The gaseous products pass through the liquid trap and into a three-necked round-bottomed flask, where gas samples were collected using a gas syringe every 30 minutes. At the end of the run, the liquid trap was emptied and the liquid samples were analyzed on a gas chromatograph equipped with a CP Wax 52 column and an FID detector. Gas samples were analysed on a gas chromatograph equipped with a CP SIL 8 column and an FID detector.

The space time yield to methyl acetate product in grams per kilogram of catalyst per hour is shown in Figure 1. As can be seen from Figure 1, after the initial catalyst conditioning period (up to 600 minutes reaction time), the catalyst is very stable for the production of methyl acetate.

### Catalysts 4 to 13

The general method for preparation of catalysts 4 to 14 is set out below.

Approximately 5 g (1.74 mmol) H₃PW₁₂O₄₀ was dissolved in 25 ml of tetrahydrofuran (THF). Approximately 5 g of silica (Grace, grade G57) was then added and the resulting mixture stirred for 2-4 hours.

A solution of a rhodium-ligand complex was formed by adding 20 ml of THF to a mixture of approximately 450 mg (1.74 mmol) dicarbonyl-acetylacetonato-rhodium(I) and approximately 1.74 mmol of the ligand. Carbon monoxide evolved from the solution.

The solution of the rhodium-ligand complex was then added to the heteropolyacid/silica mixture and the resulting mixture stirred for 15 minutes. The solvent was then removed from the mixture using a rotary evaporator. The resulting powder was dried overnight at 60°C under a stream of nitrogen. The amounts of components employed and the particular ligand used for each of catalysts 4 to 14 are shown in Table 1.

**Table 1**

| Catalyst | H₃PW₁₂O₄₀ | | Silica | Ligand | | | (acac)Rh(CO)₂ | |
|---|---|---|---|---|---|---|---|---|
| | g | mmol | g | Type | mg | mmol | mg | mmol |
| 4 | 5.008 | 1.74 | 5.009 | Xantphos | 1014.1 | 1.75 | 447.7 | 1.74 |
| 5 | 5.026 | 1.75 | 5.023 | Dpp-benz | 784.1 | 1.76 | 449.7 | 1.74 |
| 6 | 5.008 | 1.74 | 5.008 | BIPHEP | 918.6 | 1.76 | 450.1 | 1.74 |
| 7 | 5.016 | 1.74 | 5.020 | TERPHOS | 1094.0 | 1.75 | 448.1 | 1.74 |
| 8 | 5.015 | 1.74 | 5.010 | TRIPHOS | 936.3 | 1.75 | 449.3 | 1.74 |
| 9 | 5.0148 | 1.74 | 5.037 | TERPY | 410.9 | 1.76 | 449.1 | 1.74 |
| 10 | 5.019 | 1.74 | 5.011 | Dppb | 743.0 | 1.74 | 450.2 | 1.74 |
| 11 | 5.0194 | 1.74 | 5.010 | Dppp | 737.4 | 1.79 | 447.6 | 1.73 |
| 12 | 5.0303 | 1.75 | 5.019 | Dppe(F20) | 1319.5 | 1.74 | 448.3 | 1.74 |
| 13 | 5.0083 | 1.74 | 5.029 | P(oMeO-Ph)₂Ph | 569.1 | 1.77 | 450.4 | 1.75 |

### Catalysts 14 to 27

The general method for preparation of catalysts 15 to 29 is set out below.

Approximately 5 g (1.74 mmol) H₃PW₁₂O₄₀ was dissolved in 25 ml of tetrahydrofuran (THF). Approximately 5 g of silica (Grace, grade G57) was then added and the resulting mixture stirred for 2-4 hours.

A solution of a palladium-ligand complex was formed by adding 20 ml of THF to a mixture of approximately 450 mg (1.74 mmol) (CH₃CN)₂PdCl₂ and approximately 1.74 mmol of the ligand.

The solution of the palladium-ligand complex was then added to the heteropolyacid/silica mixture and the resulting mixture stirred for 15 minutes. The solvent was then removed from the mixture using a rotary evaporator. The resulting powder was dried overnight at 60°C under a stream of nitrogen.

The amounts of components employed and the particular ligand used for each of catalysts 15 to 29 are shown in Table 2.

**Table 2**

| Catalyst | H₃PW₁₂O₄₀ | | Silica | Ligand | | | (CH₃CN)₂PdCl₂ | |
|---|---|---|---|---|---|---|---|---|
| | g | mmol | g | Type | mg | mmol | mg | mmol |
| 14 | 5.0136 | 1.741 | 5.008 | Xantphos | 1014.2 | 1.753 | 449.7 | 1.733549 |
| 15 | 5.0201 | 1.743 | 5.026 | Dpp-benz | 783.8 | 1.756 | 454 | 1.750125 |
| 16 | 5.0085 | 1.739 | 5.037 | BIPHEP | 916.9 | 1.755 | 452.1 | 1.742801 |
| 17 | 5.0066 | 1.738 | 5.055 | Dpe-PHOS | 944.7 | 1.754 | 455.3 | 1.755137 |
| 18 | 5.0029 | 1.737 | 5.03 | TERPHOS | 1098.5 | 1.759 | 452.7 | 1.745114 |
| 19 | 5.0211 | 1.743 | 5.025 | TRIPHOS | 936.7 | 1.752 | 454.1 | 1.750511 |
| 20 | 5.005 | 1.738 | 5.028 | TERPY | 409.7 | 1.756 | 450.4 | 1.736248 |
| 21 | 5.0065 | 1.738 | 5.039 | BINAP | 1091.6 | 1.753 | 452.8 | 1.745499 |
| 22 | 5.0049 | 1.738 | 5.032 | Dppb | 748.8 | 1.756 | 449.7 | 1.733549 |
| 23 | 5.0122 | 1.740 | 5.026 | Dppp | 724.6 | 1.757 | 453.2 | 1.747041 |
| 24 | 5.0251 | 1.745 | 5.025 | Dppe(F20) | 1326.2 | 1.749 | 449.2 | 1.731622 |
| 25 | 5.0192 | 1.743 | 5.016 | dppe | 700.2 | 1.757 | 453.2 | 1.747041 |
| 26 | 5.0144 | 1.741 | 5.005 | P(oMeO-Ph)₂Ph | 565.6 | 1.755 | 449.7 | 1.733549 |
| 27 | 5.0074 | 1.739 | 5.019 | PPh₃ | 460.5 | 1.756 | 454.1 | 1.750511 |

### Catalysts 28 to 33

The general method for preparation of catalysts 30 to 36 is set out below.

Approximately 5 g (1.74 mmol) H₃PW₁₂O₄₀ was dissolved in 25 ml of tetrahydrofuran (THF). Approximately 5 g of silica (Grace, grade G57) was then added and the resulting mixture stirred for 2-4 hours.

A solution of an iridium-ligand complex was formed by adding 20 ml of THF to a mixture of approximately 604 mg (1.74 mmol) (acetylacetonato)Ir(CO)₂ and approximately 1.74 mmol of the ligand. Carbon monoxide evolved from the solution.

The solution of the iridium-ligand complex was then added to the heteropolyacid/silica mixture and the resulting mixture stirred for 15 minutes. The solvent was then removed from the mixture using a rotary evaporator. The resulting powder was dried overnight at 60°C under a stream of nitrogen. The amounts of components employed and the particular ligand used for each of catalysts 30 to 36 are shown in Table 3.

**Table 3**

| Catalyst | H₃PW₁₂O₄₀ | | Silica | Ligand | | | (acac)Ir(CO)₂ | |
|---|---|---|---|---|---|---|---|---|
| | g | mmol | g | Type | mg | mmol | mg | mmol |
| 28 | 5.0114 | 1.74 | 5.044 | TRIPHOS | 934.5 | 1.75 | 603.5 | 1.74 |
| 29 | 5.0055 | 1.74 | 5.026 | TERPHOS | 1094.6 | 1.75 | 604.2 | 1.74 |
| 30 | 5.0169 | 1.74 | 5.013 | Dpe-PHOS | 947.5 | 1.76 | 603.8 | 1.74 |
| 31 | 5.0092 | 1.74 | 5.032 | BIPHEP | 916.9 | 1.75 | 604.0 | 1.74 |
| 32 | 5.0059 | 1.74 | 5.015 | Dpp-Benz | 785.1 | 1.76 | 603.8 | 1.74 |
| 33 | 5.0145 | 1.74 | 5.021 | Xantphos | 1014.4 | 1.75 | 604.1 | 1.74 |

### Example 2

Catalysts 4 to 33 were tested for activity in the carbonylation of methanol according to the method described below.

0.13 ml of a 0.5 to 1 mm sieve fraction of catalyst was loaded into a sample holder of an automated high throughput testing facility. Prior to loading of the catalyst, 0.25 ml of a 250-500 µm sieve fraction of corundum was loaded into the sample holder to serve as a guard bed. The catalyst samples were heated to 225°C under an atmosphere of pure carbon monoxide. The samples remained under a pure carbon monoxide atmosphere at 225°C for 3 hours. The samples were then exposed to a mixture of carbon monoxide, methanol, nitrogen and 10% argon at a GHSV of 2000 hr⁻¹. The molar ratio of carbon monoxide : methanol: nitrogen was 2 : 1 : 2.

All of catalysts 4 to 33 were found to be active for the carbonylation of methanol to methyl acetate.

### Example 3

Catalysts 4 to 33 were tested for activity in the carbonylation of methanol in the presence of hydrogen according to the same general method used in Example 2, except that the reaction temperature was 200°C and instead of a feed gas comprising carbon monoxide, methanol, nitrogen and argon, a feed gas comprising carbon monoxide, methanol, hydrogen and argon, wherein the molar ratio of carbon monoxide : methanol : hydrogen was 2 : 1 : 2 was employed.

All of catalysts 4 to 33 were found to be active for the carbonylation of methanol to methyl acetate in the presence of hydrogen.

## Claims

1. A carbonylation process for the production of acetic acid and/or methyl acetate by contacting, in a carbonylation reactor, carbon monoxide with a feed comprising methanol and/or reactive derivative thereof in the vapour phase and in the presence of a heterogeneous catalyst comprising a heteropolyacid, wherein the heteropolyacid has been ion-exchanged or loaded with an organometallic complex of a group VIII metal and at least one ligand, which ligand comprises one or more nitrogen and/or phosphorous donor atoms.

2. A process as claimed in claim 1 wherein the heteropolyacid catalyst is represented by the formula H₃M₁₂XO₄₀ wherein M is tungsten, molybdenum, chromium, vanadium, tantalum or niobium and X is phosphorous.

3. A process as claimed in claim 1 wherein the heteropolyacid catalyst is represented by the formula H₄M₁₂XO₄₀ wherein M is tungsten, molybdenum, chromium, vanadium, tantalum or niobium and X is silicon.

4. A process as claimed in any preceding claim wherein the Group VIII metal is selected from rhodium, palladium, iridium, nickel and mixtures thereof.

5. A process as claimed in claim 4 wherein the Group VIII metal is rhodium.

6. A process as claimed in any preceding claim wherein the heteropolyacid is ion-exchanged or loaded with one or more further metals in addition to the Group VIII metal present as part of the organometallic complex.

7. A process as claimed in any preceding claim wherein the total amount of metal loaded or ion-exchanged onto the heteropolyacid is such that at least 0.5 protons are retained by the heteropolyacid.

8. A process as claimed in any preceding claim wherein the catalyst is supported on an inert support.

9. A process as claimed in claim 8 wherein the support is silica.

10. A process as claimed in claim 8 or claim 9 wherein the catalyst comprises 20 to 80% by weight based on the total weight of the catalyst and support.

11. A process as claimed in any preceding claim wherein the at least one ligand is a bidentate or tridentate diphenylphosphino-containing ligand.

12. A process as claimed in claim 11 wherein the at least one ligand is selected from dppp, dppb, dpp-Benz, BINAP, dppe, dppe(F20), TERPHOS, TRIPHOS, ANTHRAPHOS, Xantphos and DPE-phos.

13. A process as claimed in any of claims 1 to 10 wherein the at least one ligand is selected from bipy and TERPY.

14. A process as claimed in any preceding claim wherein water is present in the feed at a concentration of at least 1wt%, such as at least 2wt%.

15. A process as claimed in any preceding claim wherein the feed comprises up to 20wt% water, such as up to 15wt% water.

16. A process as claimed in any preceding claim wherein the process is carried out in the presence of hydrogen.
